# EUROPEAN PATENT APPLICATION

(11) **EP 4 678 150 A1**
(43) Date of publication of application: **14.01.2026**
(21) Application number: 24188478.2
(22) Date of filing: 12.07.2024
(51) Int. Cl.: A61F 13/15, D04H 1/425, A61F 13/53, A61F 13/531

(54) **METHOD AND APPARATUS FOR FORMING SEPARATED FIBROUS STRUCTURES**

(71) Applicant: COAX Technologies S.r.l., 26010 Monte Cremasco Cremona (IT)
(72) Inventor: ZAMPOLLO, Fabio, 26013 Crema (IT)
(74) Representative: Plischke, Manfred

(57) **Abstract**

The present invention is a process for the manufacturing of a series of separated fibrous structures of the air-laying type. Such structures may suitably be absorbent, and may be used for or - when enveloped between envelope webs - as bed-pads or for other absorbent articles.

## Description

### Field of the invention

The present invention relates to process of manufacturing a series of separated fibrous structures of the air-laying type. Such structures may suitably be absorbent, and may be used for or as bed-pads or for other absorbent articles.

### Background

Fibrous sheet materials, particularly fibrous sheet materials for absorbing fluids, are manufactured for many uses, for example they are incorporated into absorbent articles such as disposable diapers, incontinent bed-pads and catamenial napkins as fluid absorption or fluid transmission and/or diffusion elements, for example, as absorbent cores that are intended to absorb and retain body fluids.

Dry laying and, more specifically, air laying processes are widely used to produce webs from dry fibers, which can in turn be used e.g., as sheet materials for absorbing fluids. Particularly, the air laying process refers to the formation of webs with a random fiber orientation. The fibrous sheet materials produced by air laying processes are soft, flexible and porous, and are particularly suitable for use as liquid absorbent structures in absorbent articles, such as disposable diapers, sanitary napkins, pantiliners, incontinent or bed-pads, and wipes. Air laying processes are well known in the art, as are improvements aiming at evenly distributing the fibers in the airlaid structure and/or enhancing processability.

In US5527171 (Niro) the deposition of air suspended fibers in homogeneous layers is described by using multiple rows of rotating impellers in a forming head.

Similarly, US6233787 (Dan-Web / Advanced Nonwoven S/A) shows an apparatus for uniformly distributing a disintegrated material on a fiber layer forming surface by a stirrer having impellers rotating a short distance above a collecting surface.

US20030070262 (Oerlikon) describes two air-lay stations placed one after the other serving for the dry production of a non-woven fiber web, the stations including a fiber feed duct, an air laying forming head, a perforated screen as collecting surface, and a suction box for supporting the deposition of the fibers on the screen. A separate fiber source is part of each station.

Further, WO200004232 (M&J) describes the production of a nonwoven web of fibers out of a fibrous material, such as cellulose pulp. The fibrous material is disintegrated by a hammer mill and deposited at the outlet of a forming head on an endless collection wire, thereby forming a web of fibers. Nits are extracted from the forming head via a transport fan and a second air duct. Furthermore, the nits are defibrated and returned to the forming head.

Also, WO2004106604A1 (Oerlikon) aims at avoiding nits by employing a fiber distributor in a forming head at optimizing the rotational speed of the wings of the distributor.

Such production units are typically operated in an "off-line" modus, i.e., the webs produced thereon are prepared at a wide web width for interim storage in roll- or spool-form, or boxed ("festooned") and further transported to a converting place where these are fed into a process for forming articles, such as wipes, bed-pads and the like, including the cutting to size.

Such an approach induces several areas for improvement:
- homogeneity of the web is always a concern and gets more relevant when lower basis weights are aimed at.
- the fibers should be individualized as much as possible, i.e., the fibers should have little or preferably no connection to other fibers when they are laid down to form the web. Thus, it would be advantageous to obtain maximum disintegration of the fibers as supplied and to maintain the fiber disintegration along the process by avoiding aggregation of fibers or forming of nits - but all this with minimized - or even no - damage to the fibers, such as breaking up fibers, and low energy input.
- as the interim handling of the webs between web forming and web converting induces significant effort, it would be advantageous to skip such a process step, and feed the freshly formed web directly into a connected converting step, such as sandwiching the separated webs between envelope webs, such as topsheet and backsheet, thereby forming finished articles.

### Summary

In order to address such problems, the present invention is in a first aspect an equipment for forming a series of separated fibrous structures, exhibiting a machine (x-)direction, a cross (y-) direction and a height (z-)direction and comprising
- a first fibrous material supply and optionally a second fibrous material supply;
- a collection system, comprising
   ∘ a collection belt comprising a plurality of air permeable regions and blocked regions which are essentially non-permeable to air;
   ∘ belt guide rolls for operating the collection belt in a continuous loop and defining the machine direction;
- a first forming head for depositing first fluff from the first fluff supply onto the collection belt in the air permeable regions;
- a first compaction unit;
- a particulate material supply and application system with a particulate material supply and application unit;
- a second forming head for depositing second fluff from the second fluff supply onto the collection belt in the air permeable regions;
- a first suction unit to cooperate with the first forming head, a second suction unit to cooperate with the second forming head, and a further suction unit to cooperate with the particulate material supply and application system;
- a first transfer unit.

Preferably, the collection belt exhibits an essentially constant thickness across its air permeable regions and blocked regions.

In another aspect, the present invention is a process for forming a series of separated fibrous structures and comprising the steps of
A1 - providing web forming equipment exhibiting a machine (x-)direction, a cross (y-) direction and a height (z-)direction and comprising:
   - a first fibrous material supply and optionally a second fibrous material supply;
   - a collection system comprising
      - a collection belt comprising a plurality of air permeable regions and blocked regions which are preferably non-permeable to air;
      - belt guide rolls for operating the collection belt in a continuous loop and defining the machine direction;
   - a first forming head for depositing first fluff from the first fluff supply onto the collection belt in the air permeable regions;
   - a first compaction unit;
   - a particulate material supply and application system with a particulate material supply and application unit;
   - a second forming head for depositing second fluff from the first or second fluff supply onto the collection belt in the air permeable regions;
   - a first suction unit to cooperate with the first forming head, a second suction unit to cooperate with the second forming head, and a further suction unit to cooperate with the particulate material supply and application system;
   - a first transfer unit;
B- depositing in the first forming box a layer of first fibers from the first fiber supply onto the air permeable regions of the collection belt, whilst supporting the deposition by vacuum suction of the first vacuum suction system such that the cross-directionally blocked regions of the collection belt are essentially free of fluff, thereby creating first fibrous web sections;
C - transferring the first fibrous web sections to and through the first compaction unit;
D - transferring the first fibrous web sections to the particulate material application system and applying particulate material onto the first fibrous web sections, thereby preferably leaving the blocked regions of the collector belt free of particulate material, preferably supported by suction of the third vacuum suction unit,
E - transferring the first fibrous web sections with the particulate material applied thereto to and into the second forming box;
F - depositing in the second forming box a layer of second fibers from the first or the second fiber supply onto the first fibrous web sections, whereby, supported by the second vacuum suction unit,
   ∘ whereby the blocked regions of the collection belt remain essentially free of fluff thereby creating a sandwich structure of the first and the second fibrous sections with particulate material there between;
G - transferring the sandwich structure to the transfer unit,
wherein the step B to G are executed in the given order.

The process may further comprise the steps of
A2 - further providing,
   ∘ a second compaction unit comprising a nip formed between
      ▪ a first roll, preferably a patterned roll
      ▪ and a counteracting vacuum roll;
   ∘ a first envelope web supply and a first envelope web combination unit, preferably a vacuum roll;
   ∘ a second envelope web supply and a second envelope web combination unit;
   ∘ optionally a first and a second glue applicator unit, adapted to apply glue to the first or the second envelope web, respectively;
   ∘ optionally a final bonding roll;
   ∘ a finishing unit, preferably comprising a separation unit and a packaging unit;
I - transferring sandwich structure of the first and the second fibrous sections with particulate material there between from aid transfer unit towards and through the nip of the second compaction unit;
K - transferring the first envelope web from the first envelope supply unit to the first envelope combining roll, optionally applying thereby an adhesive from the first adhesive applicator;
L - transferring the compressed sandwich structure to the first envelope combining roll, thereby positioning it on the first envelope web;
M - transferring the second envelope web to the second envelope web combining roll, thereby optionally applying an adhesive from the first adhesive applicator, and further into a nip between the second envelope web combining roll and the first envelope combining roll, thereby forming enveloped separated fibrous structures;
N - optionally transferring the enveloped separated sandwich structures to a final bonding unit, preferably a melt-fusion bonding unit;
O - finishing up the enveloped separated sandwich structures by separating consecutive sandwich structures from each other and/or packaging these enveloped separated fibrous structures;
P - optionally packing it in roll-form with semi-separated separation lines between adjacent structures,
wherein steps I to P are executed in the given order subsequent to step G.

For the steps B or F, the fiber supply may be executed by individualizing the fibers from a supply in a fiber board form by a hammer mill, thereby preferably orienting the axis of the hammer mill cross-directionally. Also, for the steps B or F, the fiber supply may be executed by enhancing the fiber flow by a venturi effect restriction in the fiber supply pipe.

Even further for the steps B or F, the depositing of the fibers may be supported by a fiber distribution homogenization unit comprising multiple fiber homogenization tools, preferably of the impeller type and rotating around a vertical axis.

In step A1, the providing a first fibrous material supply (1110') and optionally a second fibrous material supply (1110") preferably includes the step of providing untreated cellulose fibers.

### Brief description of the Figures

Fig. 1 shows an equipment for operating the present invention of forming a series of separated fibrous structures.
Fig. 2 shows further elements of equipment for operating the present invention of forming a series of separated fibrous structures.

The figures are schematic only, and not to scale. Same numerals refer to same or equivalent features or elements, single (`) or multiple (", "', ...) apostrophes indicate duplicate features, such a left and right or front and back, etc.

### Detailed description

The present invention relates to a process for forming a series of separated fibrous structures comprising short fibers, such as cellulosic fibers.

Wood pulp fibers are a preferred starting material as may be formed by a variety of pulping processes, such as kraft pulp, sulfite pulp, thermomechanical pulp, and the like. Further, the wood fibers may be any high-average fiber length wood pulp, low-average fiber length wood pulp, or mixtures of the same. One example of suitable high-average length wood pulp fibers includes softwood fibers such as, but not limited to, northern softwood, southern softwood, redwood, red cedar, hemlock, pine (e.g., southern pines), spruce (e.g., black spruce), combinations thereof, and the like. One example of suitable low-average length wood pulp fibers includes hardwood fibers, such as, but not limited to, eucalyptus, maple, birch, aspen, and the like. The fibers may be treated so as to allow optimization of processing and or absorbency properties, The term "treated" as used herein is understood to include any means of introducing the additive to the fiber and/or fibrous matrix, but not limited to, such as coating, spraying, printing, chemical modifications, wet-end additions applications to the fibers as well as blending untreated fibers with treated fibers. Moreover, if desired, secondary fibers obtained from recycled materials may be used, such as fiber pulp from sources such as, for example, newsprint, reclaimed paperboard, and office waste, or recycled diapers, be it from factory scrap or be it post-consumer recycling.

The fibrous material may be delivered as fibrous board or in fibrous sheet form. Also bales of fibrous material may be delivered as may be opened prior to be used in the process according to the present invention. Further, "roughly graded material" may be employed, wherein fibers are present as clusters of several hundreds up to several thousand fibers in the roughly graded material. It is an object of the present invention to dry lay, or air lay, such fibrous material to form fibrous webs for being converted into articles comprising such fibrous webs.

Such articles comprise further materials, typically web materials for stabilizing and/or containing the fibrous webs. Conventional air-laying processes are often operated in an "off-line" modus, whereby jumbo reels of the fibrous structure are produced on machines with a large width. Such wide roll may be cut to a lower width and rewound for being transferred to a converter, where the fibrous materials are cut to the appropriate size for being introduced into articles, often absorbent articles, such as wipes, or pads like bed-pads, or the like.

Whilst such processes allow efficient production of the fibrous webs, rewinding and transport imply process complexity and cost. When avoiding this by producing airlaid webs in-line on the converting process, conventional air-laying processes provide continuous webs that imply potential for contamination when being cut to the right length, and further require cut and space operation to feed into the finished articles.

In a first aspect, the present invention is an air-laying process producing a series of separated fibrous webs for being processed in further processing steps for forming articles comprising fibrous structures.

In another aspect, the present invention is a process for converting airlaid fibrous structures into a series of articles, whereby fibrous structures are separated be a region which is free of the airlaid fibrous structure, but essentially only comprises envelope webs, such as a backsheet, and a cover web such as a topsheet. In a particular execution, the airlaid fibrous structures are made up of two airlaid webs, preferably differing in density, and further optionally comprising particulate material therebetween, such as superabsorbent polymers (SAP) in particulate or fibrous form. However, in contrast to conventional structures, the ones as made according to the present invention do not require, and are preferably free of, a supporting substrate, such as typically tissues, that not only enable a conventional continuous production but also prevent SAP particles to give a rough feel for the backsheet side and may even damage backsheets. Similarly, conventional structures also comprise a user-oriented preformed cover web, often a paper tissue, for preventing SAP particles to penetrate through to user's skin. Thus, such backsheet side and topsheet side tissues may be readily omitted when sandwiching the SAP between in-line formed airlaid layers.

In another aspect the present invention is an equipment to produce such separated airlaid webs. It is an important element of such an equipment that the collecting surface on which the fibers are forming the fibrous web exhibit regions of varying air permeabilities, preferably regions with and regions without or at least significantly reduced air permeability.

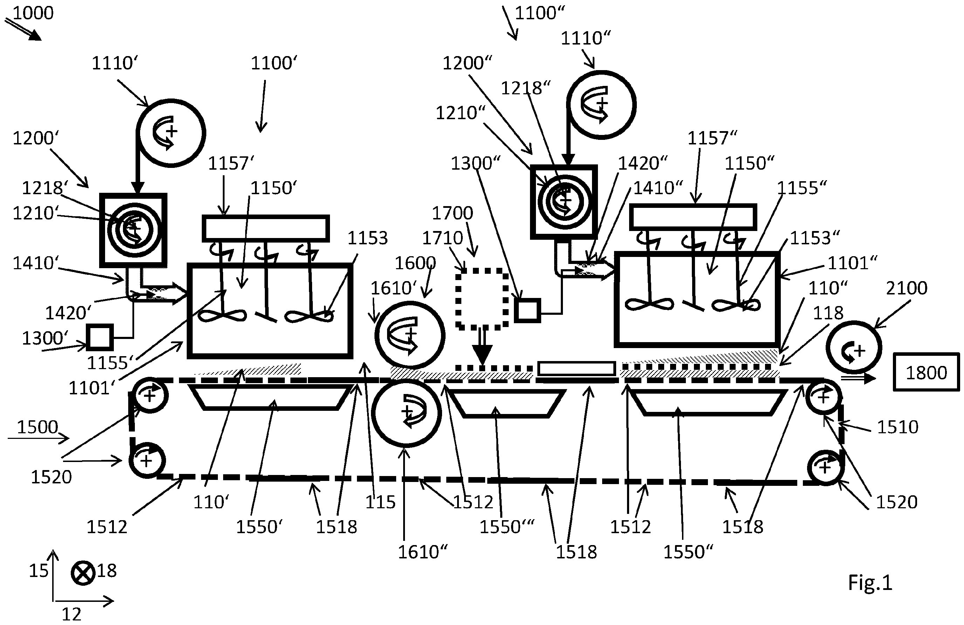

In order to further explain the present invention, reference is made to Fig. 1, which however, should not be seen to limit the present invention.

An equipment 1000 for forming a series of fibrous airlaid webs 110 comprises at least one web forming head 1100' and may comprises a further web forming head 1100". These may be conventional air-laying heads, as known from the prior art as discussed in the above. As indicated in the Fig 1, the web forming heads 1100', 1100" may be essentially identical in design, and even may be operated at identical process conditions, but these could also have differing designs and process settings.

The equipment exhibits an overall process or machine (x-) direction 12, a width or cross-machine direction (y-) 18, and a height (z-) direction 15 perpendicular to both and aligned with gravity. As exemplarily shown in Fig. 1, the fibrous material is delivered from a fiber supply 1110', 1110" towards a disintegration unit 1200', 1200"

In a particular execution, the fiber supply 1110', 1110" is in the form of a compressed fiber board roll, and the disintegration unit is a hammer mill 1210, and preferably the rotating axis of the hammer mill is along the width or y-direction of the overall equipment. However, other fiber supply options may be employed, such as using fiber bales, from which fibers may be individualized by picking teeth.

The fibers are transferred, preferably by pneumatic transfer in piping 1410, preferably further aided by additional fiber supply air 1300 by a venturi effect restriction 1420.

The forming heads 1100, 1100" may comprise a forming box 1101', 1101" with one or more openings 1102', 1102", respectively, for receiving the individualized fibers and an outlet 1108', 1108" through which the individualized fibers are deposited. The forming heads preferably comprise a fiber distribution homogenization unit 1150', 1150" for homogenizing the x-y-directional distribution of the fibers.

In a preferred execution the fiber distribution homogenization units 1150', 1150" comprise a multiplicity of fans 1153', 1153" rotating by means of drives 1157', 1157" around vertical axes 1155. As indicated in the cross-sectional view of Fig.1 three such fans 1153 are shown in each of the forming heads 1100', 1100", but - as indicated in the background section - there could be an array of fans, with two, three, four or even more arranged in the cross-directional rows and two, or four, or six, or eight or ten or even more arranged in machine-directional columns. Optionally, the fans in adjacent rows may be cross-directionally off-set, or the rows and columns are in an angled position relative to the machine direction. The drives 1157', 1157" for the fans may be a single one connected to the fans by belts or gear wheels, or multiple drives (not shown).

The individualized and x-y-directionally evenly distributed fibers are deposited though the forming box outlets 1108', 1108" onto the collection belt 1510 of the collection system 1500, further guided by first (1550') and second (1550") vacuum suction systems, respectively. It is an important aspect of the present invention that the collection belt 1510 comprises alternating regions with high air permeability 1512 and low, or preferably no air permeability 1518. Thus, the machine-directional distribution of the fibers is modified, as the air-entrained fibers are directed within the forming heads 1100', 1100" with the air stream towards the high permeability regions 1512, there forming fibrous web sections 110', 110" with machine directionally increasing thickness but with fiber free regions 115 between neighbouring web sections.

The low permeability regions 1518 may be manufactured by a multitude of approaches, such as by applying a blocking onto the surface of the belt such as fixing a strip of solid material, or applying a blocking material such as glue or silicones. Also, when the collection belt is manufactured of a thermoplastic material, the low permeability regions may be achieved by compressing the material in these regions, thereby forming air impermeable film like structures.

However, as the collection belt is run over the various guide rolls and through compression nips (as will be discussed herein below), it is particularly preferred execution that the collection belt exhibits an essentially equal thickness in the permeable (1512) and less permeable (1518) regions. Such impermeable regions may be created by impregnating the collection belt in the relevant regions with a curable resin, as well known in the art, see e.g., WO2020/256714 (P&G), to which express reference is made for the description of formation of the airflow blocking structures 1518.

The equipment 1000 further comprises a compaction unit 1600 positioned between the first (1100') and the second (1100") forming head and adapted to compress the web sections 110', indicated by to counterrotating rolls 1610, 1620, each preferably with a smooth surface thus creating a flat surface of the web sections.

Further, at least one particulate material application system 1700 comprising a particulate material supply 1710, is positioned preferably between the compaction unit 1600 and the second forming head 1100", adapted to apply particulate material, as may be SAP polymers, such as SAP particles, onto the first formed web section 110'. There is no particular limitation to the selection of such a particular material applications system. Exemplary executions include continuous or discontinuous rotary or slot applicators, optionally supported by doctor blades. Other systems include powder spray systems. For particular applications, it may be preferred to apply the particles in a pattern, such as may by well-known particle printing processes. It is highly preferred that the regions between the web sections also remain free of particulate material, e.g., via the patterned application tools. When the particles are applied in a continuous manner, the airflow blocking structures 1518 may also prevent the particles from depositing there. If this is not sufficient, particle removal tools (not shown) may be employed, such as blowing air or brushing particles away.

The equipment further comprises a web section removal unit 2100 adapted to receive the composite sandwich structures composed of web sections 110', 118, 110" and transferring these by means of a pick-up and transfer unit 2100, preferably a vacuum drum, to further web processing units 1800. A particular execution of a web processing unit is an enveloping unit for sandwiching the separated web pieces between envelope webs, such as a cover material or a topsheet, as may be a liquid and air permeable web, such as a nonwoven, and a carrier material or backsheet, such as a liquid impermeable web, e.g. a film material.

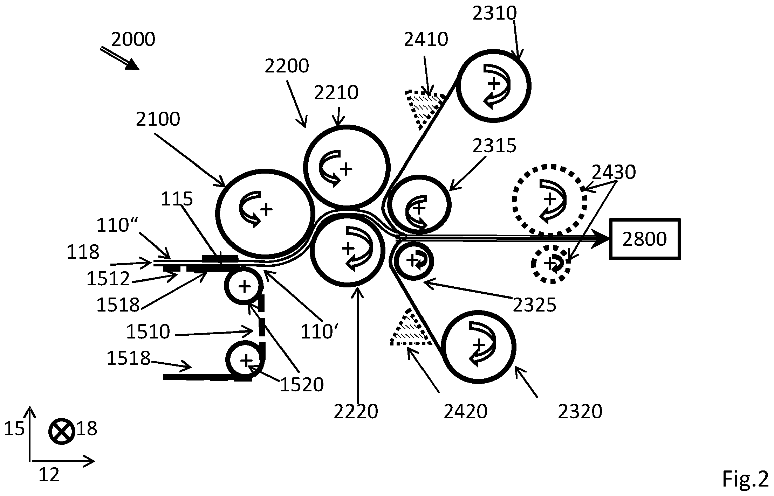

Such a further processing unit for executing further processing steps is indicated in Fig.2, comprising a compaction system 2200 for further compacting the combined web. Such a compaction may be executed in a multitude of known alternatives, such as by feeding web separated web sections into a nip between an embossing roll 2210, preferably a patterned embossing roll, and a counteracting vacuum roll 2220, preferably with a smooth, perforated surface. After the embossing, a first envelope web, as may be referred to as a topsheet, may be unwound from a first envelope supply 2310 and fed towards a first envelope web combining roll 2315, preferably also a vacuum roll. As the width of this web material is wider and the pitch of this material is longer than the separated web sections, a perimeter free of the fibrous and particulate material is formed. Whilst the web sections and the first enveloping web material are hold on the first envelope web combining roll 2315, a second enveloping web is provided from a second enveloping web supply 2320 and combined with the first enveloping web and the web sections positioned thereon, thus forming a series of individualized fibrous structures 100, separated by fiber and particle free regions 115. The structure may be held together by conventional means, such as by adhesive as may be applied to the first (2410) and/or second (2420) envelope web, or by melt-fusion bonding applied in a further processing step 2430, these options being indicated in Fig. 2 with dotted lines.

The individualized fibrous webs may then be further transferred to further processing units 2800, as may be a finishing and packaging unit, optionally with a separation unit to separate the individualized structures in the fiber and particle free regions 115.

The process is now executed on an equipment as described in the above by the following steps:
- disintegrating the fibers in a disintegration unit 1200' of a first forming head 1100'. In a preferred execution, the disintegration unit 1200' is arranged such that the individualized fibers are moved along the machine direction towards the forming head 1100' by positioning the rotating axis of the disintegration unit 1200' cross-directionally.
- feeding the individualized fibers pneumatically via connection pipe 1410' and preferably with additional air of the air supply 1300' via a venturi effect flow path restriction 1420' towards an opening 1102' of the first forming head 1100'.
- homogenizing the x-y-directional distribution of the fibers in the first forming head 1100' by the first fiber distribution homogenization system 1150'.
- depositing the fibers through the forming box outlet 1108 onto the air permeable regions 1502 of the collection belt 1510, thereby supported by the first vacuum suction system 1550' and thus forming a series of first fibrous web sections 110', separated by essentially fiber free regions 115, thereby executing the separation such that a cross-directionally extending region extending over the full width of the fibrous sections.
- transferring the series of first fibrous web sections 110' towards a first compaction unit 1600 and compressing the first fibrous web sections 110'.
- transferring the compressed first fibrous web section 110' to the particulate material application unit 1700 and applying the particulate material 118 onto the first fibrous web sections 110'. Due to the vacuum suction unit 1550'", the regions being essentially free of fibers remains also essentially free of particulate material.
- transferring the first fibrous web sections 110', with the particulate material 118 towards a second forming head 1100", and applying second fibrous web sections 110" in analogy to forming the first fibrous web section 110' as described in the above. The material selections and the process settings may be, but do not need to be, the same as for the first forming head 1100'.
- transferring the series of separated composite web sections comprising the first (110') and the second (110") web sections and the particulate material 118 sandwiched therebetween, to pick-up and transfer system 2100 and removing the composite web sections from the transfer belt 1510, preferably by applying vacuum suction in a transfer roll with a foraminous surface. The series of composite web sections may then further be transferred to further web handling units 1800.

In a particular execution, the process further comprises the following further steps for enveloping the series of separated composite web sections:
- transferring the series of separated composite sections by the pick-up and transfer system 2100 into a second compaction unit 2200, and compressing it, preferably by applying an embossing pattern of a patterned embossing roll acting 2210 against a vacuum suction roll 2220.
- enveloping the series of composite web sections between a first and a second envelope web by
   ∘ winding a first envelope web from a first envelope supply unit 2310 and optionally applying an adhesive by a first adhesive applicator 2410 on a surface of this web, which is intended to contact the composite web sections.
   ∘ combining the first envelope web and the series of composite web sections by means of a first envelop web combining unit 2315, preferably supported by vacuum, thereby separating the series of composite web sections from the second compaction unit.
   ∘ unwinding a second envelope web from a second envelope web supply unit 2320 and optionally applying an adhesive by a second glue applicator 2420.
   ∘ combining the second envelope web with and the series of composite web sections, which are positioned on the first envelope web, by means of a second envelope web combining unit 2325.
   ∘ passing the combined first and second envelope web and the series of composite web sections sandwich therebetween through a nip formed by the first (2315) and the second (2325) envelope combining units.
- Transferring the sandwich structure to a finishing unit, such as separating the series of composite web sections from each other in the fiber and particle free regions 115 of the envelope webs.

The present invention is particularly suitable for being integrated into a converting process for forming articles, such as bed-pads, or wipes, or absorbent articles, as it obviates the need of processing the "jumbo roll" webs of conventional air-laying equipment and transporting the preformed webs to the article forming converter lines.

Thus, it is a particular effect of the present invention, that separated air-laid web section may be directly fed into a further equipment units and processing steps for manufacturing articles comprising such webs sections, such as without limitation bed-pads, or wipes, or feminine hygiene pads, or other absorbent articles like diapers or adult incontinence articles.

Particular articles that may be readily manufactured according to the present invention are bed-pads, that may exhibit
- an overall article length of more than about 300 mm, or more than about 600 mm, or more than about 800 mm, but typically less than about 1500 mm.
- an overall article width of more than about 100 mm, or more than about 250 mm, or more than about 400 mm, but typically less than about 1000 mm.
- a basis weight of the sandwich structure of more than about 30 g/m² or more than about 50 g/m² or more than about 80 g/m, but typically less than about 300 g/m².
- a weight ratio of the particulate material relative to the total fiber weight in the sandwich structure of more than about 5 w-%, or more than about 10 w-%, or more than about 20 w-%, or more than about 30 w-%, or more than about 50 w-%. Or even more than about 60 w-%

In a preferred design, the article consists essentially of the sandwich structure, a topsheet, a backsheet and adhesives, whereby the latter may be absent in case of thermo-fusion bonding, such as by heat-, pressure-, or ultrasonic bonding. A particular example for such an article may be a bed-pad, exhibiting
- an overall article length of 900 mm and width of 600 mm;
- an absorbent structure length of 830 mm and a width of 530 mm;
- an overall weight of the absorbent structure of about 50 g with about 17,5 g of pulp as a first (lower or backsheet side) layer, SAP weight of about 15 g, and about 17,5 g of pulp as a second (upper, topsheet side) layer.

It should be noted that the current invention is very flexible to adjusting the width of the formed webs independently of the type of fiber supply. Thus, the width of the fiber supply may be less than about 90 %, or less than about 70 %, or less than about 50 % or less than about 30 % of the width of the resulting web 100. In an exemplary execution, the fibers were supplied from a fiber board roll of a width of 500 mm, but can be converted into a web having a width of 830 mm - a suitable size for forming, e.g., bed-pads.

## Claims

**1.** An equipment for forming a series of separated fibrous structures (100), exhibiting a machine (x-)direction (12), a cross (y-) direction (18) and a height (z-)direction (15) and comprising
- a first fibrous material supply (1110') and optionally a second fibrous material supply (1110");
- a collection system (1500), comprising
∘ a collection belt (1510) comprising a plurality of air permeable regions (1512) and blocked regions (1518) which are essentially non-permeable to air;
∘ belt guide rolls (1520) for operating said collection belt (1510) in a continuous loop and defining said machine direction (12);
- a first forming head (1100') for depositing first fluff from said first fluff supply (1110') onto said collection belt (1510) in the air permeable regions (1512);
- a first compaction unit (1600);
- a particulate material supply and application system (1700) with a particulate material supply and application unit (1710);
- a second forming head (1100") for depositing second fluff from said second fluff supply (1110") onto said collection belt (1510) in the air permeable regions (1512);
- a first suction unit (1550') to cooperate with said first forming head (1100'), a second suction unit (1550") to cooperate with said second forming head (1100"), and a further suction unit (1550‴) to cooperate with said particulate material supply and application system;
- a first transfer unit (2100).

**2.** An equipment for forming a series of separated fibrous structures (100) according to claim 1, whereby said collection belt (1510) exhibits an essentially constant thickness across its air permeable regions (1512) and blocked regions (1518).

**3.** A process for forming a series of separated fibrous structures (100), comprising the steps of A1 - providing web forming equipment exhibiting a machine (x-)direction (12), a cross (y-) direction (18) and a height (z-)direction (15) and comprising:
- a first fibrous material supply (1110') and optionally a second fibrous material supply (1110");
- a collection system (1500), comprising
- a collection belt (1510) comprising a plurality of air permeable regions (1512) and blocked regions (1518) which are preferably non-permeable to air;
- belt guide rolls (1520) for operating said collection belt (1510) in a continuous loop and defining the machine direction;
- a first forming head (1100') for depositing first fluff from said first fluff supply (1110') onto said collection belt (1510) in the air permeable regions (1512);
- a first compaction unit (1600);
- a particulate material supply and application system (1700) with a particulate material supply and application unit (1710);
- a second forming head (1100") for depositing second fluff from said first (1110') or second fluff supply (1110") onto said collection belt (1510) in the air permeable regions (1512);
- a first suction unit (1550') to cooperate with said first forming head (1100'), a second suction unit (1550") to cooperate with said second forming head (1100"), and a further suction unit (1550‴) to cooperate with said particulate material supply and application system;
- a first transfer unit (2100);
B- depositing in said first forming box (1100') a layer of first fibers from said first fiber supply (1110') onto the air permeable regions (1512) of said collection belt (1510), whilst supporting the deposition by vacuum suction of said first vacuum suction system (1550') such that said cross-directionally blocked regions (1518) of said collection belt (1510) are essentially free of fluff, thereby creating first fibrous web sections (110');
C - transferring said first fibrous web sections (110') to and through said first compaction unit (1600);
D - transferring said first fibrous web sections (110') to said particulate material application system (1700) and applying particulate material onto the first fibrous web sections (110'), thereby preferably leaving said blocked regions (1518) of said collector belt (1510) free of particulate material, preferably supported by suction of said third vacuum suction unit (1550‴),
E - transferring said first fibrous web sections (110') with said particulate material applied thereto to and into said second forming box (1100");
F - depositing in said second forming box (1100") a layer of second fibers from said first (1110') or said second (1110") fiber supply onto said first fibrous web sections (110'), whereby, supported by said second vacuum suction unit (1550"),
∘ whereby said blocked regions (1518) of said collection belt (1510) remain essentially free of fluff,
thereby creating a sandwich structure of said first (110') and said second (110") fibrous sections with particulate material (118) there between;
G - transferring said sandwich structure to said transfer unit (2100),
wherein said step B to H are executed in the given order.;

**3.** A process according to claim 3, further comprising the steps of
A2 - further providing,
∘ a second compaction unit (2200) comprising a nip formed between
▪ a first roll, preferably a patterned roll (2210)
▪ and a counteracting vacuum roll (2220);
∘ a first envelope web supply (2310) and a first envelope web combination unit (2315), preferably a vacuum roll;
∘ a second envelope web supply (2320) and a second envelope web combination unit (2325);
∘ optionally a first (2410) and a second glue applicator unit, adapted to apply glue to said first or said second envelope web, respectively;
∘ optionally a final bonding roll (2430);
∘ a finishing unit, preferably comprising a separation unit and a packaging unit;
H - transferring sandwich structure of said first (110') and said second (110") fibrous sections with particulate material (118) there between from aid transfer unit (2100) towards and through said nip of said second compaction unit (2200);
I - transferring said first envelope web from said first envelope supply unit (2310) to said first envelope combining roll (2315), optionally applying thereby an adhesive from said first adhesive applicator (2410);
K - transferring said compressed sandwich structure to said first envelope combining roll (2315), thereby positioning in on said first envelope web.
L - transferring said second envelope web to said second envelope web combining roll (2325), thereby optionally applying an adhesive from said first adhesive applicator (2420), and further into a nip between said second envelope web combining roll (2325) and said first envelope combining roll (2315), thereby forming enveloped separated fibrous structures (100);
M - optionally transferring said enveloped separated sandwich structures to a final bonding unit (2430), preferably a melt-fusion bonding unit;
N - finishing up said enveloped separated sandwich structures by separating consecutive sandwich structures from each other and/or packaging these enveloped separated fibrous structures
O - optionally packing it in roll-form with semi-separated separation lines between adjacent structures,
wherein steps I to O are executed in the given order subsequent to step H.

**5.** A process according to claim 3 or 4, wherein in steps B or F, the fiber supply is executed by individualizing said fibers from a supply in a fiber board form by a hammer mill, thereby preferably orienting the axis of the hammer mill cross-directionally.

**6.** A process according to any of claim 3 to 5, wherein in steps B or F, the fiber supply is executed by enhancing the fiber flow by a venturi effect restriction in the fiber supply pipe.

**7.** A process according to any of claim 3 to 6, wherein in steps B or F, said depositing of said fibers is supported by a fiber distribution homogenization unit (1150) comprising multiple fiber homogenization tools (1153), preferably of the impeller type rotating around a vertical axis.

**8.** A process according to any of claim 3 to 7, wherein in step A1 said step of providing a first fibrous material supply (1110') and optionally a second fibrous material supply (1110") includes the step of providing untreated cellulose fibers.
